# EUROPEAN PATENT SPECIFICATION

(11) **EP 4 398 975 B1**
(45) Date of publication and mention of the grant of the patent: **05.11.2025**
(21) Application number: 22840299.6
(22) Date of filing: 08.12.2022
(51) Int. Cl.: A61N 1/04, A61N 1/36

(54) **COMPRESSION GARMENT ASSEMBLY FOR APPLYING TTFIELDS**
KOMPRESSIONSKLEIDUNGSANORDNUNG ZUR ANWENDUNG VON TUMORBEHANDLUNGSFELDERN
ENSEMBLE VÊTEMENT DE COMPRESSION POUR L'APPLICATION DE CHAMPS DE TRAITEMENT DE TUMEUR

(30) Priority: 10.12.2021 US 202163288229 P
(43) Date of publication of application: 17.07.2024
(73) Proprietor: Novocure GmbH, 6340 Baar (CH)
(72) Inventor: SMITH-PETERSEN, Kathryn, Portsmouth, New Hampshire 03801 (US)
(74) Representative: Boden, Keith McMurray
(86) International application number: PCT/IB2022/061945
(87) International publication number: WO 2023/105468

(56) References cited:
- US-A1- 2010 312 307
- US-A1- 2019 298 987
- US-A1- 2021 187 289
- US-A1- 2021 212 634

## Description

### BACKGROUND

TTFields therapy is a proven approach for treating tumors. For example, using the Optune^{®} system for delivering tumor treating fields (i.e., TTFields), the TTFields are delivered to patients via four transducer arrays placed on the patient's skin in close proximity to a tumor. The transducer arrays are arranged in two pairs, and each transducer array is connected via a multiwire cable to an electric field generator. The electric field generator (a) sends an AC current through one pair of arrays during a first period of time; then (b) sends an AC current through the other pair of arrays during a second period of time; then repeats steps (a) and (b) for the duration of the treatment.

US-A-2010/0312307 discloses an electrotherapy apparatus facilitating accurate and repeatable application to a neuromuscular area to be treated by a patient, which comprises a flexible garment adapted to be worn by the patient, and stimulation electrodes are attachable to the body-facing surface of the garment on at least a predetermined portion(s) thereof.

### SUMMARY OF THE INVENTION

It is desirable to assist a patient in placing and maintaining the transducer arrays at predetermined locations on the patient's body. It is to such a system that the present disclosure is directed.

The present invention provides a garment according to claim 1.

Embodiments of the invention are disclosed in the dependent claims.

The details of one or more implementations of the subject matter described in this specification are set forth in the accompanying drawings and the description below. Other aspects, features and advantages of the subject matter will become apparent from the description, the drawings, and the claims.

### BRIEF DESCRIPTION OF THE DRAWINGS

The accompanying drawings, which are incorporated in and constitute a part of this specification, illustrate one or more implementations described herein and, together with the description, explain these implementations. The drawings are not intended to be drawn to scale, and certain features and certain views of the figures may be shown exaggerated, to scale or in schematic in the interest of clarity and conciseness. Not every component may be labeled in every drawing. Like reference numerals in the figures may represent and refer to the same or similar element or function. In the drawings:
FIG. 1A is a front perspective view of an exemplary embodiment of a garment constructed in accordance with the present disclosure.
FIG. 1B is a rear perspective view of an exemplary embodiment of the garment of FIG. 1A constructed in accordance with the present disclosure.
FIG. 2A is a perspective view of an exemplary embodiment of a back portion of a garment constructed in accordance with the present disclosure.
FIG. 2B is a front view of an exemplary embodiment of a transducer array constructed in accordance with the present disclosure.
FIG. 3A is a partial cross-sectional view of an exemplary embodiment of a back portion of a garment receiving a transducer array constructed in accordance with the present disclosure.
FIG. 3B is a partial cross-sectional view of another exemplary embodiment of the back portion of the garment receiving a transducer array constructed in accordance with the present disclosure.
FIG. 3C is a perspective front view of exemplary components constructed in accordance with the present disclosure.
FIG. 4 is a rear perspective view of an exemplary embodiment of the garment engaged with a transducer array constructed in accordance with the present disclosure.
FIG. 5 is a process flow chart of an exemplary method in accordance with the present disclosure.

### DETAILED DESCRIPTION

TTFields are generally delivered to patients via four transducer arrays placed on the patient's skin conventionally as two orthogonal pairs in locations chosen to best target the tumor (target region). Each transducer array is configured as a set of coupled electrode elements (for example, about 2 cm in diameter) that are interconnected via flex wires. When placing the arrays on the patient, the medical gel adheres to the contours of the patient's skin and ensures good electrical contact of the device with the body.

Before explaining at least one embodiment of the inventive concept(s) in detail by way of exemplary language and results, it is to be understood that the inventive concept(s) is not limited in its application to the details of construction and the arrangement of the components set forth in the following description. The inventive concept(s) is capable of other embodiments or of being practiced or carried out in various ways. As such, the language used herein is intended to be given the broadest possible scope and meaning; and the embodiments are meant to be exemplary - not exhaustive. Also, it is to be understood that the phraseology and terminology employed herein is for the purpose of description and should not be regarded as limiting.

Unless otherwise defined herein, scientific and technical terms used in connection with the presently disclosed inventive concept(s) shall have the meanings that are commonly understood by those of ordinary skill in the art. Further, unless otherwise required by context, singular terms shall include pluralities and plural terms shall include the singular.

All of the compositions, assemblies, systems, kits, and/or methods disclosed herein can be made and executed without undue experimentation in light of the present disclosure. While the compositions, assemblies, systems, kits, and methods have been described in terms of particular embodiments, it will be apparent to those of skill in the art that variations falling under the scope of the claims may be made.

Headings are provided for convenience only and are not to be construed to limit the invention in any manner. Embodiments illustrated under any heading or in any portion of the disclosure may be combined with embodiments illustrated under the same or any other heading or other portion of the disclosure. Any combination of the elements described herein in all possible variations thereof is encompassed by the disclosure unless otherwise indicated herein or otherwise clearly contradicted by context.

As utilized in accordance with the present disclosure, the following terms, unless otherwise indicated, shall be understood to have the following meanings:
The use of the term "a" or "an" when used in conjunction with the term "comprising" in the claims and/or the specification may mean "one," but it is also consistent with the meaning of "one or more," "at least one," and "one or more than one." As such, the terms "a," "an," and "the" include plural referents unless the context clearly indicates otherwise. Thus, for example, reference to "a compound" may refer to one or more compounds, two or more compounds, or greater numbers of compounds. The term "plurality" refers to "two or more."

The use of the term "at least one" will be understood to include one as well as any quantity more than one. The use of ordinal number terminology (i.e., "first," "second," "third," "fourth," etc.) is solely for the purpose of differentiating between two or more items and is not meant to imply any sequence or order or importance to one item over another or any order of addition, for example.

As used herein, any reference to "one embodiment," "an embodiment," "some embodiments," "one example," "for example," or "an example" means that a particular element, feature, structure, or characteristic described in connection with the embodiment is included in at least one embodiment. The appearance of the phrase "in some embodiments" or "one example" in various places in the specification is not necessarily all referring to the same embodiment, for example.

As used in this specification and claim(s), the words "comprising" (and any form of comprising, such as "comprise" and "comprises"), "having" (and any form of having, such as "have" and "has"), "including" (and any form of including, such as "includes" and "include"), or "containing" (and any form of containing, such as "contains" and "contain") are inclusive or open-ended and do not exclude additional, unrecited elements or method steps.

The term "patient" as used herein encompasses any mammals including human and veterinary subjects. "Mammal" for purposes of treatment refers to any animal classified as a mammal, including (but not limited to) humans, domestic and farm animals, nonhuman primates, and any other animal that has mammary tissue. In some embodiments, the term "patient" may apply to a simulation mannequin for use in teaching, for example.

Circuitry, as used herein, may be analog and/or digital components, or one or more suitably programmed processors (e.g., microprocessors) and associated hardware and software, or hardwired logic. Also, "components" may perform one or more functions. The term "component," may include hardware, such as a processor (e.g., microprocessor), an application specific integrated circuit (ASIC), a field programmable gate array (FPGA), a combination of hardware and software, and/or the like. The term "processor" as used herein means a single processor or multiple processors working independently or together to perform a task.

The term "TTField", as used herein, means tumor treating field. TTFields are low intensity (e.g., 1-4 V/cm) alternating electric fields of medium frequencies (about 50 kHz to about 1 MHz, and more preferable from about 50 kHz to about 500 kHz) that when applied to a conductive medium, such as a human body, via electrodes may be used, for example, to treat tumors as described in U.S. Patents Nos. 7,016,725, 7,089,054, 7,333,852, 7,565,205, 7,805,201, and 8,244,345 by Palti and in the publication "Disruption of Cancer Cell Replication by Alternating Electric Fields", Cancer Res. 2004 64:3288-3295, Eilon D. Kirson, et al. TTFields have been shown to have the capability to specifically affect cancer cells and serve, among other uses, for treating cancer. TTFields therapy is an approved mono-treatment for recurrent glioblastoma (GBM), and an approved combination therapy with chemotherapy for newly diagnosed GBM patients.

The term "transducer array", as used herein, means a conductive transducer array or a non-conductive transducer array. Exemplary transducer arrays may include, for example, pads disclosed in any one of U.S. Patent Publication No. 2021/0346693 entitled "CONDUCTIVE PAD GENERATING TUMOR TREATING FIELD AND METHODS OF PRODUCTION AND USE THEREOF" and U.S. Patent Publication No. 2022/0193404 entitled "OPTIMIZATION OF COMPOSITE ELECTRODE".

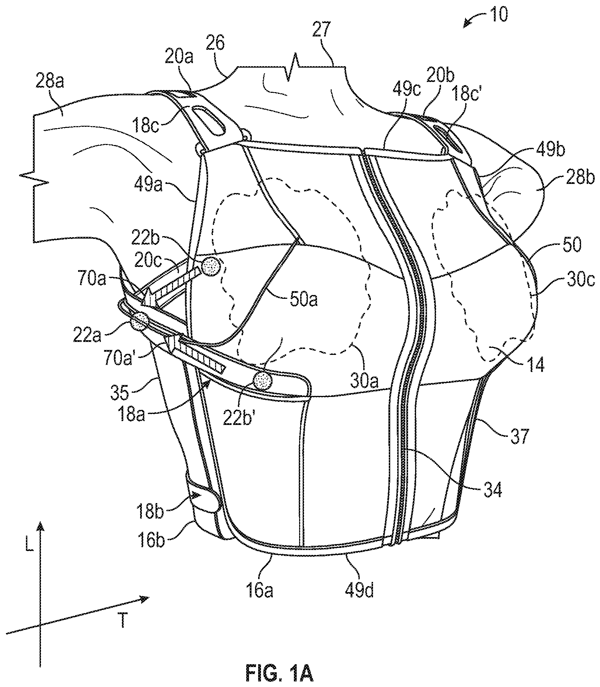

Referring now to the drawings, and in particular FIG. 1A, shown therein is a front perspective view of an exemplary embodiment of a garment 10 constructed in accordance with the present disclosure. The garment 10 generally comprises a support layer 14, a first adjustment assembly 18a, and two or more connection points 22a-n, referred to generally as connection points 22 and individually as connection point 22, are illustrated in FIG. 1A as first connection point 22a and second connection point 22b. Though the connection points 22a-n are referred to herein as points for explanatory purposes, it will be understood that the connection points 22an may have a length, a width, and an area. In some embodiments, one or more of the connection points 22a-n may have one or more openings.

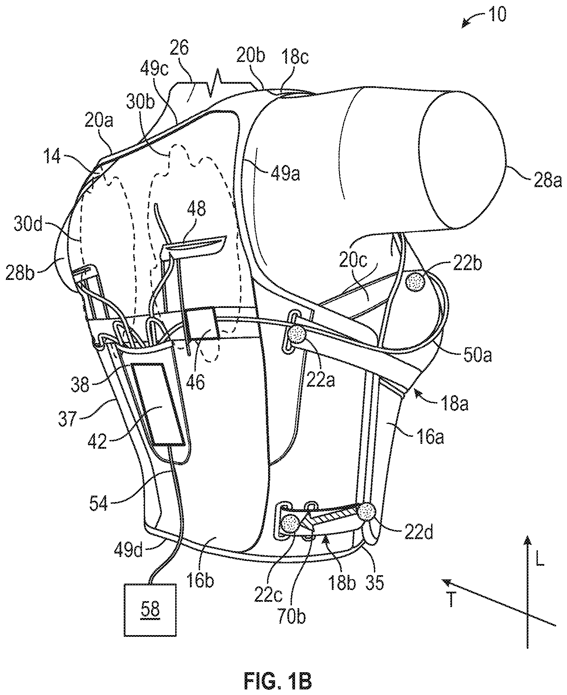

As shown in FIG. 1A, the garment 10 is shown in use by a patient 26 whereby the support layer 14 supports one or more transducer array 30a-n, referred to generally as transducer arrays 30 and individually as transducer array 30, over a treatment area of the patient 26. In this example, the garment 10 supports first, second, third, and fourth transducer arrays 30a, 30b, 30c, 30d, with the first and third transducer arrays 30a, 30c located on a front portion 16a of the garment 10 configured to be on a front of the patient 26 in use, and the second and fourth transducer arrays 30b, 30d located on a back portion 16b of the garment 10 configured to be on a back of the patient 26 in use (FIG. 1B).

In one embodiment, the garment 10 is configured to cover the torso of the patient 26 when in use, as shown in FIG. 1A. In this embodiment, the garment 10 may be one or more of an undergarment, a shirt, tank-top, jacket, vest, sweater, and/or the like. In other embodiments, the garment 10 may be configured to cover a different part of the patient 26, such as, one or more of the patient's head, arm, waist, leg, hand, or foot. For example, the garment 10 may be a head covering (such as a hat), an arm covering (such as a sleeve), a hand covering (such as a glove), a foot covering (such as a sock or shoe), a leg or waist covering (such as pants, shorts, stockings), or the like.

Throughout this application, the garment 10 may be described as being a vest, vest-like, or having features of a vest, however, the garment 10 is not limited to being a vest, as described above. In other embodiments, the garment 10 may be a girdle or a shoulder strap/brace, for example.

In one embodiment, the garment 10 includes at least two anchor points 20a-n associated with the support layer 14. Each anchor point 20 may be associated with a particular location on the patient 26 where the garment 10 is in contact with the patient 26 such that when a force is applied between the two anchor points, the garment 10 is substantially maintained at the particular location. The anchor points 20a-n may be configured relative to the garment 10 such that, in use, the anchor points 20a-n are positioned in the same place relative to the patient 26 when the garment 10 is taken off and put back on. The anchor points 20a-n may be configured to maintain the position of the garment relative to the patient when in use. In one implementation, for example, one or more of the adjustment assemblies 18, e.g., adjustment assemblies 18a-c shown in FIGS. 1A-1B, may be or may include an anchor point. In one implementation, for example, one or more of the connection points 22 may be, or may include, an anchor point.

In one embodiment, the anchor point(s) 20 of the garment 10 may be associated with a corresponding position on the patient where the contours of the patient's body would limit the garment 10 from substantial movement, such as, for example, at the intersections between the patient's neck and shoulders, the patient's armpits, the patient's hips, and/or the patient's groin areas. In one embodiment, the anchor points 20 are opposing anchor points, such as on opposite sides of the patient's body, e.g., a right armpit anchor point 20c and a left-side-of-neck second neck anchor point 20b, a groin anchor point and a first and second neck anchor point 20a, 20b, which may correspond to positions on either side of the neck of the patient 26, or the like.

In one embodiment, the anchor points 20 may be selected such that movement of the garment 10 is minimized when in use. For example, the garment 10 may be a girdle wherein the anchor points 20 of the support layer 14 engage the patient's hips to limit the garment 10 from moving.

In one embodiment, the garment 10 is "breathable", that is, the garment 10 includes one or more perforation configured to allow air to move through the garment 10 to the patient 26, such as to contact the patient's skin, for example. In some embodiments, the garment 10 may be "wicking", that is, the garment 10 may be constructed to allow sweat or moisture near the patient's skin to be removed, or wicked, from the patient 26 through the garment 10.

In one embodiment, the garment 10 may be worn under outer clothing by the patient 26. For example, the garment 10 may be an undergarment and the patient 26 may wear a shirt over the garment 10. In this embodiment, the garment 10 may be considered form-fitting, such that when the patient 26 wears a shirt over the garment 10, a third-party viewing the patient is unlikely to notice that the patient 26 is wearing the garment 10, thereby providing the patient 26 privacy of treatment while using the garment 10. Such privacy may have benefits for the patient 26, such as providing the patient 26 with confidence to continue with normal, daily activities during treatment without concern of third-parties noticing the treatment.

The support layer 14 is configured to be worn by the patient 26 and may comprise the front portion 16a and the back portion 16b of the garment 10. The support layer 14 is sized and dimensioned to cover at least a portion of the patient 26, preferably in a form-fitting manner. Further, the support layer 14 is configured to support the two or more connection points 22a-n. In one embodiment, the support layer 14 is constructed of a breathable material as described above, and may be, for example, constructed of a woven material (e.g., a woven fabric), a mesh material, or the like. In other embodiments, the support layer 14 may be constructed of a non-woven material (e.g., a non-woven fabric).

In one embodiment, the support layer 14 may be considered form-fitting, that is, the support layer 14 may be configured to conform to the patient 26 when the patient is wearing the garment 10.

In one embodiment, the support layer 14 covers the torso of the patient 26, as shown in FIG. 1A. In this embodiment, the support layer 14 may be one or more of an undergarment, a shirt, tank-top, jacket, vest, sweater, and/or the like. In other embodiments, the support layer 14 covers a different part of the patient 26, such as, one or more of the patient's head, arm, waist, leg, hand, or foot. For example, the support layer 14 may be a head covering (such as a hat), an arm covering (such as a sleeve), a hand covering (such as a glove), a foot covering (such as a sock or shoe), a leg or waist covering (such as pants, shorts, stockings), or the like.

Throughout this application, the support layer 14 may be described as being a vest, vest-like, or having features of a vest, however, the support layer 14 is not limited to being a vest, as described above.

In one embodiment, the support layer 14 is "breathable", that is, the support layer 14 includes one or more perforation configured to allow air to move through the support layer 14 to the patient 26, such as to contact the patient's skin, for example. In other embodiments, the support layer 14 may be "wicking", that is, the support layer 14 may be constructed to allow sweat or moisture near the patient's skin to be removed, or wicked, from the patient 26 through the support layer 14.

In one embodiment, as shown in FIG. 1A, the support layer 14 may be configured to be worn under outer clothing by the patient 26. For example, the support layer 14 may be an undergarment and the patient 26 may wear a shirt over the support layer 14. In this embodiment, the support layer 14 may be considered form-fitting, such that when the patient 26 wears a shirt over the support layer 14, a third-party viewing the patient is unlikely to notice that the patient 26 is wearing the support layer 14, thereby providing the patient 26 privacy of treatment while using the garment 10. Such privacy may have benefits for the patient 26, such as providing the patient 26 with confidence to continue with normal, daily activities during treatment without concern of third-parties noticing the treatment.

In some embodiments, a first portion of the support layer 14 and a second portion of the support layer 14 are constructed of either different materials or of the same material exhibiting differing properties.

In one embodiment, the first adjustment assembly 18a is adjustably secured to the support layer 14 at the first connection point 22a on the back portion 16b and the second connection point 22b on the front portion 16a such that adjustment of the first adjustment assembly 18a varies a spacing between the first connection point 22a and the second connection point 22b thereby varying a tension of the support layer 14 in at least a portion of the treatment area. In one embodiment, the first adjustment assembly 18a may be adjustable between a first position exerting a first force 70a in a direction extending from the second connection point 22b to the first connection point 22a, and a second position.

In one embodiment, the adjustment assembly 18 may be further adjustably secured at a third connection point 22b' on the front portion 16a such that adjustment of the first adjustment assembly 18a varies the spacing between the third connection point 22b' and one or more of the second connection point 22b and the first connection point 22a, thereby further varying the tension of the support layer 14 in at least the portion of the treatment area. In one embodiment, the first adjustment assembly 18a is adjustable between the first position and the second position thereby exerting the first force 70a in the direction extending from the second connection point 22b to the first connection point 22a and exerting a second force 70a' in a direction extending from the third connection point 22b' to the second connection point 22b. By applying force to the support layer 14, the support layer 14 presses against the transducer arrays 30, such as the first transducer array 30a, thereby assisting in maintaining the sufficient electrical connection between the transducer array 30a and the patient's body. The sufficient electrical connection between the transducer array 30a and the patient's body may be measured as the impedance between the first and second transducer arrays 30a, 30b.

Each connection point 22 may be associated with the support layer 14 and one or more of the adjustment assemblies 18. In one embodiment, the first connection point 22a may be at a fixed location in or on the back portion 16b of the support layer 14 at a first vertical position along a longitudinal axis L and a first horizontal position along a transverse axis T, whereas the second connection point 22b may be at an adjustable location in or on the front portion 16a of the support layer 14 at a second vertical position along the longitudinal axis L and a second horizontal position along the transverse axis T where the second vertical position is vertically displaced along the longitudinal axis L from the first vertical position and the second horizontal position is horizontally displaced along the transverse axis T from the first horizontal position.

In one embodiment, as shown in FIG. 1A, the first adjustment assembly 18a may be a strap extending between the front portion 16a to the back portion 16b of the support layer 14, e.g., between the first connection point 22a and the second connection point 22b. The first connection point 22a may be at a fixed location on the back portion 16b of the support layer 14. For example, an end of the strap may be sewn to the support layer 14. In other embodiments, the second connection point 22b may be a first component of a hook and loop fastener while a second component of the hook and loop fastener is integrated with the strap. The strap, therefore, may be adjustably attached to the first component of the hook and loop fastener. The first adjustment assembly 18a is shown in FIG. 1A in a first position.

As described above, a patient 26 is any mammal including a human or a veterinary subject. The patient 26 may be any mammal; however, the patient 26 will be referred to as a human and having human properties. It is understood, however, that the patient 26 may be any mammal and that the garment 10 may be configured to be worn by, or placed on, a patient 26 that is other than a human.

The one or more transducer array 30a-n, such as shown in FIG. 1A as the first transducer array 30a, may a field-generating transducer array, such as one of the transducer arrays incorporated above. The transducer arrays 30 may be configured to generate a TTField when connected to an electric field generator such as the electric field generator described in one or more of U.S. Patents Nos. 7,016,725, 7,089,054, 7,333,852, 7,565,205, 7,805,201, and 8,244,345 by Palti.

In one embodiment, the garment 10 may further include a fastening component 34. The fastening component 34 may be configured to allow the patient 26 to remove and/or re-attach the garment 10 without adjustment of the one or more adjustment assemblies 18. In one embodiment, in order to adjustably secure the one or more adjustment assemblies 18 to the support layer 14, the fastening component 34 is first engaged thereby ensuring that adjustment of the one or more adjustment assemblies 18 is made from a known configuration, thus enabling the patient 26 to take off/remove the garment 10 and put on/apply the garment 10 while maintaining tension of the support layer 14 over at least a portion of the treatment area when the garment 10 is reapplied.

In one embodiment, as shown in FIG. 1A, the fastening component 34 is a zipper bisecting the support layer 14 in a direction parallel with the longitudinal axis L and passing over or near the patient's sternum when the garment 10 is worn by the patient. In some embodiments, the fastening component 34 may be otherwise constructed or have additional fastener(s), such as with a plurality of buttons and button-holes, one or more hook and loop fastener, and/or the like.

Referring now to FIG. 1B, shown therein is a rear perspective view of an exemplary embodiment of the garment 10 constructed in accordance with the present disclosure. In the example shown in FIG. 1B, the garment 10 includes the first adjustment assembly 18a associated with the support layer 14 and the first connection point 22a on the back portion 16b of the support layer 14.

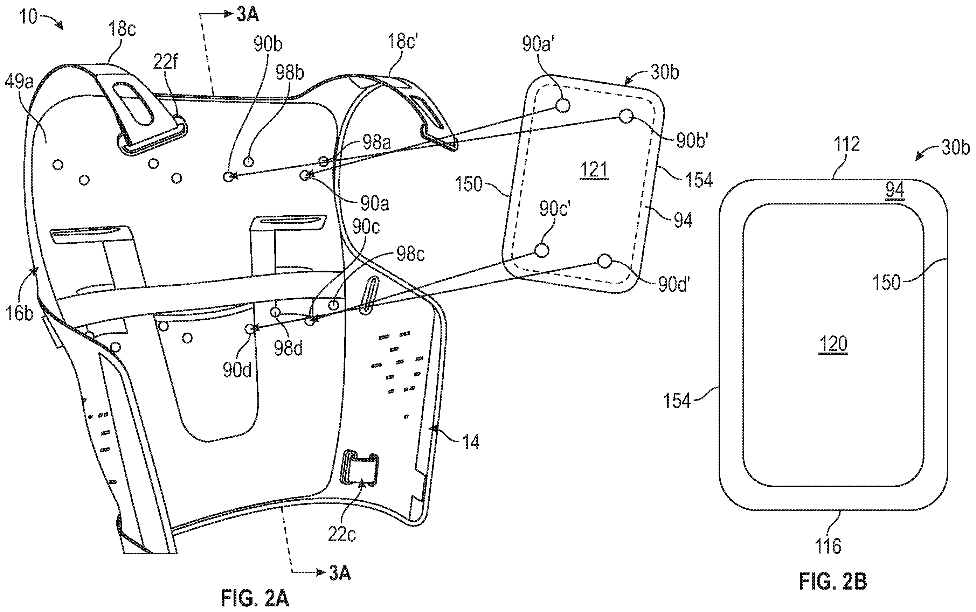

In one embodiment, the garment 10 is provided with a second adjustment assembly 18b adjustably secured to the support layer 14 at a fourth connection point 22c and a fifth connection point 22d such that adjustment of the second adjustment assembly 18b varies a spacing between the fourth connection point 22c and the fifth connection point 22d thereby varying a tension of the support layer 14 (such as between the front portion 16a and the back portion and/or relative to the patient 26). In one embodiment, the second adjustment assembly 18b may be adjustable between a first position exerting a third force 70b (shown in FIG. 2) in a direction extending from the fifth connection point 22d to the fourth connection point 22c, and a second position. In one embodiment, the second adjustment assembly 18b may be a waist strap.

The fourth connection point 22c and the fifth connection point 22d may be different connection points 22 than the first connection point 22a and the second connection point 22b. In one embodiment, the fourth connection point 22c and the fifth connection point 22d are vertically displaced in the longitudinal axis L from the first vertical position of the first connection point 22a and the second vertical position of the second connection point 22b, respectively. The fifth connection point 22d may further be attached to the front portion 16a of the support layer 14 at a fourth horizontal position horizontally displaced along the transverse axis T from a third horizontal position of the fourth connection point 22c and the first horizontal position of the first connection point 22a. In one embodiment, the fourth connection point 22c and the fifth connection point 22d may be vertically displaced below (towards the direction of the waist) from the first connection point 22a and the second connection point 22b on the back portion 16b and the front portion 16a, respectively.

In one embodiment, the garment 10 further includes the second transducer array 30b adjustably affixed to the support layer 14 as described in more detail below in reference to FIG. 2.

In one embodiment, as shown in FIG. 1B, the garment 10 may further include a hub support 38 configured to carry a hub 42. The hub support 38 may be formed as a pocket integrated with the support layer 14 positioned in the back portion 16b of the support layer 14 configured to cover a middle of the patient's back, for example. The hub support 38 may be a pocket operable to receive the hub 42. The hub support 38 may be formed of the same or different material as the support layer 14 and, in some embodiment, may be formed of breathable material to provide ventilation for the hub 42. Further, in one embodiment, the hub support 38 may extend over a portion of the support layer 14 that further includes a hub support backing attached thereto.

In one embodiment, as shown in FIG. 1B, the garment 10 may further include a lead guide 46 positioned on the back portion 16b of the support layer 14 configured to cover a mid-region on the patient's back. The lead guide 46 may be a loop of material configured to receive and guide one or more lead 50a-n, referred to generally as leads 50 and individually as lead 50, from transducer arrays 30 to the hub 42. A lead 50a may extend from the transducer array 30, through the lead guide 46 and connect to the hub 42. In one embodiment, the support layer 14 includes one or more aperture 48 through which the leads 50 may pass through the support layer 14.

Further shown in FIG. 1B is a cable 54 extending from, and electrically connecting, the hub 42 to an electric field generator 58. In one embodiment, the hub 42 is electrically coupled to each transducer array 30 via a separate one of the lead 50 and electrically coupled to the electric field generator 58, thereby communicably coupling the electric field generator 58 to one of more of the transducer array 30 enabling the electric field generator 58 to transmit one or more signal to the transducer arrays 30 causing the transducer arrays 30 to generate the TTField. The transducer arrays 30, leads 50, hub 42, cable 54, and electric field generator 58 may be constructed as described in U.S. Patent Publication No. 2022/0096819 entitled "CONNECTOR FOR DETACHABLE ARRAY".

In one embodiment, the garment 10 has a right side 35 and a left side 37, the left side 37 opposite the right side 35 across an approximately medial line parallel to the longitudinal axis L, and one or more of the first, second, third, fourth, and/or fifth connection point 22a, 22b, 22b', 22c, 22d, and/or the first adjustment assembly 18a and the second adjustment assembly 18b are duplicated on both the right side 35 and the left side 37 of the garment 10.

Referring to FIGS. 1A-1B, generally, the adjustment assemblies 18 and connection points 22a-n are shown as being on the right-hand side of the garment 10 (and of the patient 26 when in use), however, it should be understood that the adjustment assemblies 18 and connection points 22a-n are not limited to being on the right side 35 of the garment 10 and may be on the left side 37 instead of, or in addition to, being on the right side 35. In other words, while FIGS. 1A-1B are shown as having the adjustment assemblies 18 on the right-hand side of the garment 10 (and of the patient 26, when in use), additional adjustment assemblies 18 may be placed at similar, mirrored locations, or other locations, on the lefthand side of the garment 10 (and of the patient 26, when in use). For example, in one implementation, the number of adjustment assemblies 18 on the garment 10 may include six adjustment assemblies 18, including three adjustment assemblies 18 on the left side 37 and three adjustment assemblies 18 on the right side 35.

Additionally, the garment 10 may have a third adjustment assembly 18c. While only three adjustment assemblies 18 are shown on the right side 35 of the garment 10, additional adjustment assemblies 18 (such as a fourth adjustment assembly 18c' shown in FIG. 1A) may be included on the garment 10 to vary additional connection points 22 on the support layer 14 resulting in additional forces 70 being applied to the support layer 14 to apply pressure to the transducer array(s) 30.

Further, as shown in FIGS. 1A-1B, the garment 10 may include a first aperture 49a, a second aperture 49b, a third aperture 49c, and a fourth aperture 49d. The garment 10 may be configured such that, when in use, the patient's right arm 28a and the patient's left arm 28b extend through the garment 10 and the support layer 14 via the first aperture 49a and the second aperture 49b, respectively. Further, the garment 10 may be configured such that, when in use, the neck 27 of the patient 26 may extend through the garment 10 and the support layer 14 via the third aperture 49c. In one embodiment as shown, each aperture 49 is bound by one or more of the support layer 14, the front portion 16a, the back portion 16b, and the adjustment assemblies 18. For example, the first aperture 49a may be bound by the front portion 16a, the back portion 16b, the third adjustment assembly 18c, and the first adjustment assembly 18a. The third aperture 49c may be bound by the front portion 16a, the back portion 16b, the third adjustment assembly 18c and the fourth adjustment assembly 18c'. The fourth aperture 49d may be bound by the front portion 16a and the back portion 16b. It should be understood that other configurations of the garment 10, the support layer 14, and the adjustment assemblies 18 may be used to form apertures 49 different from the first, second, third, and fourth apertures 49a-d shown in FIGS. 1A-1B to receive other parts of the patient 26. For example, the support layer 14 could be provided with two apertures to receive legs of the patient 26.

In one embodiment, where the garment 10 and the support layer 14 are constructed of a contiguous material, and each aperture 49 may be bound by the support layer 14 on all sides.

Further, the garment 10 may include additional apertures 49 not configured to receive a part of the patient 26. For example, if the patient 26 does not have the right arm 28a, the garment 10 may still include the first aperture 49a, however, the first aperture 49a may not receive a part of the patient 26, or, alternatively, the first aperture 49a may be positioned, e.g., via the adjustment assemblies 18, to secure against the patient's shoulder.

In one embodiment, one or more aperture 49 is not bound by the front portion 16a, the back portion 16b, the third adjustment assembly 18c, and the first adjustment assembly 18a on all sides of the aperture 49 as described below.

Referring now to FIG. 2, shown therein is an illustration of an exemplary embodiment of the back portion 16b of the garment 10 constructed in accordance with the present disclosure. The garment 10 may include one or more transducer array fastener 90a-n, collectively transducer array fasteners 90 and singly transducer array fastener 90. The garment 10 shown in FIG. 2, includes transducer array fasteners 90a-d positioned on a skin-facing surface 80 of the back portion 16b of the garment.

In one embodiment, each transducer array fastener 90 is attached to the support layer 14 at a predetermined location. The predetermined location may generally be selected such that an extent of the area bound by the location of each transducer array fastener 90 substantially covers a treatment area of the patient 26. While the garment 10 is shown as including only first, second, third, and fourth transducer array fasteners 90a, 90b, 90c, 90d, it is understood that the garment 10 could include a greater or lesser number of transducer array fasteners 90.

Shown in FIG. 2A and 2B is the second transducer array 30b. The second transducer array 30b has a top edge 112 and a bottom edge 116. The second transducer array may have a skin-facing surface 120 opposing a support-layer surface 121, as well as a first side 150 and a second side 154. The second transducer array 30b may include one or more complementary array fastener 90', shown as complementary array fasteners 90a'-d', on the opposed support-layer surface 121 of the second transducer array 30b, and operable to be coupled to corresponding ones of the transducer array fasteners 90. For example, as depicted in FIG. 2, first, second, third, and fourth complementary array fasteners 90a', 90b', 90c', 90d' are operable to be coupled to the first, second, third, and fourth transducer array fasteners 90a, 90b, 90c, 90d, respectively.

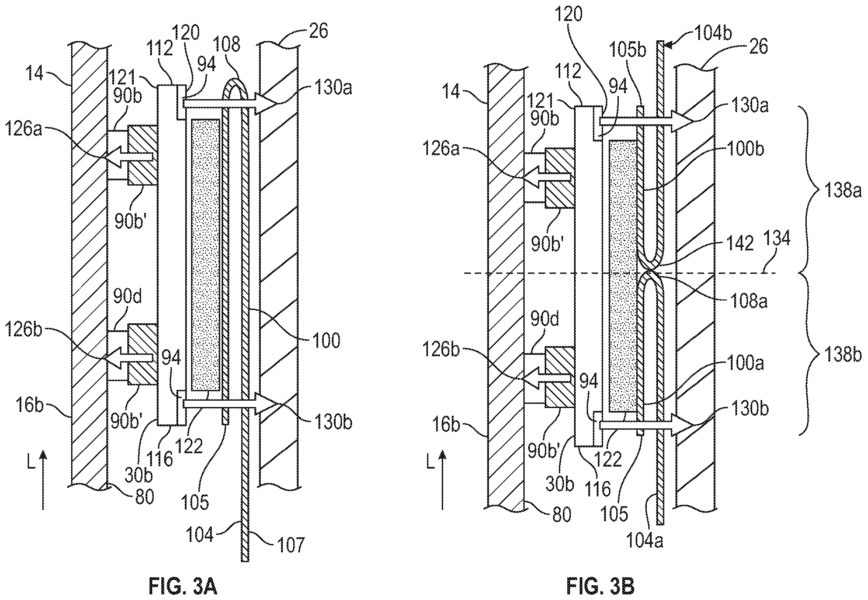

In one embodiment, the second transducer array 30b, and in some embodiments, other transducer arrays 30, further include an attaching member 94. The attaching member 94 is shown as an edge region on the skin-facing surface 120 of the transducer array (described in more detail in reference to FIG. 3A-3B below) of the transducer array 30b. It should be noted that while the attaching member 94 is shown as in the edge region of the transducer array 30b, the attaching member 94 may be more than one attaching member 94 and may be located at any location on the skin-facing surface 120 of the transducer array 30b.

A pair of corresponding transducer array fastener 90 and complementary array fastener 90', may be referred to as a fastener set. The fastener set may be constructed of any fastener that can hold and support the second transducer array 30b to the back portion 16b of the garment 10. For example, each fastener set may comprise one or more of a hook and loop fastener, snap fastener, tape, double-sided tape, and/or the like.

In one embodiment, the garment 10 may further include one or more second transducer array fasteners 98a-n, depicted as first, second, third, and fourth second transducer array fasteners 98a-d. The second transducer array fasteners 98 may be operable to be coupled to corresponding ones of the complementary array fasteners 90'. For example, the first, second, third, and fourth second transducer array fastener 98 may be operable to be coupled to the first, second, third, and fourth complementary array fasteners 90a', 90b', 90c', and 90d', respectively. The second transducer array fasteners 98may be offset in a range from about one quarter inch to about one half inch from the transducer array fasteners 90. The offset may be along the longitudinal axis L, the transverse axis T, or along a combination of both the longitudinal axis L and the transverse axis T. In some embodiments, the second transducer array fasteners 98 are offset dependent on the size of the target region. For example, in a torso of the patient, the second transducer array fasteners 98 may be offset in a range from about two inches to about three inches from the transducer array fasteners 90.

By attaching the second transducer array fasteners 98 at a different predetermined location on the support layer 14 as the transducer array fasteners 90, the patient 26 can easily move the second transducer array 30b from a first position, where the complementary array fasteners 90' are coupled to the transducer array fasteners 90, to a second position where the complementary array fasteners 90' are coupled to the second transducer array fasteners 98. The second transducer array 30b in the first position may be positioned over an overlapping yet different treatment area as the second transducer array 30b in the second position. In other embodiments, the treatment area of the second transducer array 30b in the first position may coincide with at least a portion of the treatment area of the second transducer array 30b in the second position.

In some embodiments, the treatment area of the second transducer array 30b in the first position and the treatment area of the second transducer array 30b in the second position are both selected to target the same target area within the patient 26; however, in other embodiments, the treatment area of the second transducer array 30b in the first position and the treatment area of the second transducer array 30b in the second position are selected to target different target areas within the patient 26.

In one embodiment, one or more aperture 49, e.g., the first aperture 49a as shown in FIG. 2, is not bound by the front portion 16a, the back portion 16b, the third adjustment assembly 18c, and the first adjustment assembly 18a on all sides of the first aperture 49a. For example, the garment 10 may comprise the back portion 16b without the front portion 16a, similar to the garment 10 shown in FIG. 2. In this embodiment, the third and fourth adjustment assemblies 18c, 18c' may be constructed of a non-flexible material or a material resistant to deformations. Here, the garment 10 may be applied to the patient 26 by positioning an arm of the patient 26 through the first aperture 49a and drawing the back portion 16b against a back of the patient, thereby applying the second transducer array 30b connected to the garment 10, i.e., via the transducer array fasteners 90 and the complementary array fasteners 90', to the patient 26 over a treatment area of a target region.

Referring now to FIG. 3A, shown therein is a cross-sectional diagram of an exemplary embodiment of the second transducer array 30b having a protective layer 100 disposed against second the transducer array 30b along the longitudinal axis L. As shown in FIG. 3A, the garment 10 has been applied to the patient 26 thereby placing the protective layer 100 against the patient's skin.

As shown in FIG. 3A, the support layer 14 is shown with the second and fourth transducer array fasteners 90b,90d connected to the second and fourth complementary array fasteners 90b', 90d', respectively. The second and fourth complementary array fasteners 90b', 90d' are shown as fixed to the second transducer array 30b. The protective layer 100 may be applied to the skin-facing surface 120 of the second transducer array 30b.

In some embodiments, the skin-facing surface 120 may be composed of, or at least partially coated with, a medical gel, such as medical gel layer 122. In these embodiments, the medical gel layer 122 may be disposed between the skin-facing surface 120 and the protective layer 100.

In one embodiment, the protective layer 100 may cover at least a portion of the second transducer array 30b that when removed, such as by the patient 26, adheres the second transducer array 30b to the patient's skin on or over the target area after the garment 10 is placed onto the patient 26.

In one embodiment, the protective layer 100 is configured to protect the second transducer array 30b and the medical gel layer 122 from potential damage, such as inadvertent adhesion to itself or other objects prior to the garment 10 being placed onto the patient 26. Further, the protective layer 100 may be configured to protect the medical gel layer 122 from loss of adhesion due to exposure moisture loss of the medical gel layer 122. The protective layer 100 may be removed (such as by the patient 26) after the garment 10 is placed onto the patient 26, but before the second transducer array 30b receives an electrical signal from the electric field generator 58. In one embodiment, the protective layer 100 may be an electric insulator covered by a non-stick coating, such that the protective layer 100 prevents or substantially reduces accidental application of a TTField to the patient 26 before the second transducer array 30b is attached to the patient 26.

In one embodiment, an adhesive may be used between the second transducer array 30b and the protective layer 100 such that application of the second transducer array 30b, having the protective layer 100 already applied thereto, to the support layer 14 may be performed without the protective layer 100 separating from the second transducer array 30b.

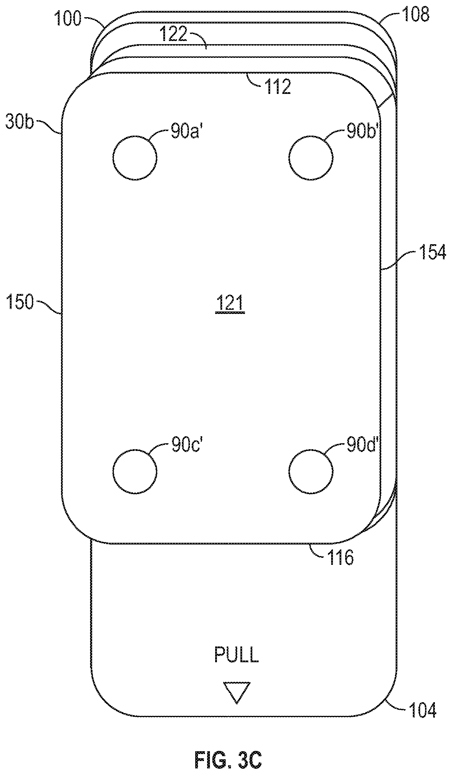

In one embodiment, the protective layer 100 may have a first end 105 in contact with and/or covering the medical gel layer 122, and a second end 108 that curves in a direction away from the first end 105. The protective layer 100 may have, or be connected to, a release member 104, which is connected to the second end 108 and which may be positioned parallel to the first end 105. In one embodiment, the release member 104 may extend past an outer edge of the support layer 14, such as along the longitudinal axis L past bottom edge 116. In one embodiment, the protective layer 100 and the release member 104 may be contiguous and formed of the same, flexible, material. In one embodiment, the protective layer 100, and/or the release member 104, are formed of a non-conductive material. In one embodiment, the release member 104 and the protective layer 100 may form a U-shape or a J-shape. In one embodiment, the release member 104 is positioned adjacent to the first end 105 of the protective layer 100. In one embodiment, at least a portion of the release member 104 is positioned adjacent to the first end 105 of the protective layer 100. While the second end 108 of the protective layer 100 is shown positioned proximate to the top edge 112 of the second transducer array 30b in FIG. 3C, it should be understood that the second end 108 may be positioned and/or have an orientation offset from or otherwise different from the position or orientation of the top edge 112. In one embodiment, the release member 104 has a skin-facing surface 107.

In one embodiment, when the patient 26, or a third-party, pulls the release member 104, such as in an opposing direction of the second end 108 of the protective layer 100, the release member 104, attached to the protective layer 100, disengages the protective layer 100 from the second transducer array 30b, thereby causing the second transducer array 30b and/or the medical gel layer 122 to come into contact with the patient's skin. As shown in FIG. 3A, as a force is exerted on the release member 104 in a downward direction along the longitudinal axis L, the protective layer 100 at the second end 108 is caused to peel away from the second transducer array 30b and/or the medical gel layer 122, followed by the first end 105 of the protective layer 100 being caused to peel away from the second transducer array 30b and/or the medical gel layer 122.

Also shown in FIG. 3A is a fastener force 126 (shown as first fastener force 126a and second fastener force 126b) exerted on the second transducer array 30b when a force is applied to the support layer 14 to pull the support layer 14 away from the patient 26, thereby causing the transducer array fasteners 90 to disengage from the complementary array fasteners 90'. Further shown is an attaching force 130 (shown as first attaching force 130a and second attaching force 130b) exerted on the second transducer array 30b towards the patient 26 when the force is applied to the support layer 14 due to the attaching member 94. In other words, as the force is applied to the support layer 14 to remove the support layer 14 from the patient 26, the force is transferred through the transducer array fastener 90 engaged to the complementary array fastener 90', the second transducer array 30b, and the attaching member 94 to the patient 26. As the force applied is increased, the fastener forces 126 and attaching forces 130 also increase.

As depicted, the first attaching force 130a is greater than the first fastener force 126a and the second attaching force 130b is greater than the second fastener force 126b. Thus, when the force applied to pull the support layer 14 away from the patient 26 is approximate to the fastener force 126, the transducer array fasteners 90 will disengage from the complementary array fasteners 90' while the second transducer array 30b maintains placement on the patient 26, due to the attaching force 130 exceeding the fastener force 126. In this manner, the garment 10 can be used as an applicator for applying one or more of the transducer arrays 30a-n to the patient 26. In the example shown, the garment 10 can be used as an applicator to precisely position the second and third transducer arrays 30b, 30c to the back of the patient 26.

In one embodiment, the attaching member 94 may be selected such that the attaching force 130 of the attaching member 94 when applied to the patient 26 is greater than that fastener force 126 between the transducer array fastener 90 and the complementary array fastener 90'. In one embodiment, a sum of the attaching forces 130 is greater than a sum of the fastener forces 126. In one embodiment, the transducer array fastener 90 and the complementary array fastener 90' are chosen such that the fastener force 126 is lesser than the attaching force 130.

In one embodiment, the attaching member 94 may be an adhesive or a cohesive. For example, the attaching member 94 may be an adhesive attached to the transducer array 30b during manufacturing or during use. The protective layer 100 may be constructed from a material that is non-reactive or have a low reaction to the attaching member 94 such that the protective layer 100 protects the attaching member 94 from damage and such that when the release member 104 is engaged, e.g., by the patient 26, the protective layer 100 disengages from the attaching member 94 without damaging the attaching member 94. When the protective layer 100 disengages from the second transducer array 30b, the attaching member 94 may be exposed to the patient 26, e.g., to the skin of the patient 26, and may cause the second transducer array 30b to attach to the patient 26.

In one embodiment, the attaching member 94 may be a cohesive having a first cohesive component and a second cohesive component, wherein the first cohesive component is attached to the second transducer array 30b during manufacturing or during use. The protective layer 100 may be constructed from a material that is non-reactive or have a low reaction to the first cohesive component of the attaching member 94 such that the protective layer 100 protects the first cohesive component of the attaching member 94 from damage and such that when the release member 104 is engaged, e.g., by the patient 26, the protective layer 100 disengages from the first cohesive component of the attaching member 94 without damaging the first cohesive component. The second cohesive component may be applied to the patient 26 before applying the support layer 14 or may be applied on the skin-facing surface 107 of the release member 104 such that when the support layer 14 is applied to the patient 26, the second cohesive component on the skin-facing surface 107 of the release member 104 is transferred onto the patient 26. When the protective layer 100 disengages from the second transducer array 30b, the first cohesive component of the attaching member 94 may be exposed to the second cohesive component on the patient 26, thereby causing the second transducer array 30b to attach to the patient 26.

Referring now to FIG. 3B, shown therein is a cross-sectional diagram of an exemplary embodiment of the second transducer array 30b having a first protective layer 100a and a second protective layer 100b disposed against the second transducer array 30b and being co-planar along the longitudinal axis L. The first protective layer 100a and the second protective layer 100b may be constructed similar to the protective layer 100 of FIG. 3A except as described below.

In one embodiment, the first protective layer 100a and the second protective layer 100b are applied to the skin-facing surface 120, or to the medical gel layer 122, of the second transducer array 30b. Here, a second end 108a of the first protective layer 100a does not extend beyond the top edge 112 of the second transducer array 30b, but extends to a midline 134 different from the top edge 112 and the bottom edge 116 covering a first portion 138a of the second transducer array 30b. Further, the second protective layer 100b is inversed relative to the first protective layer 100a. For example, the first protective layer 100a may extend upwards from the bottom edge 116 towards the midline 134 of the second transducer array 30b such that the second end 108a of the first protective layer 100a is at the midline 134, and the second protective layer 100b extends downward from the top edge 112 to the midline 134 thereby covering a second portion 138b of the second transducer array 30b such that a bottom 142 of the second protective layer 100b is at the midline 134.

In this embodiment, a first release member 104a may thus attach to the second end 108a of the first protective layer 100a and extend downward as described with regards to the release mem ber 104. The first release member 104a and the first protective layer 100a may form a U-shape or a J-shape. In one embodiment, at least a portion of the first release member 104a is positioned adjacent to the first end 105 of the first protective layer 100a. A second release member 104b may attach to the bottom 142 of the second protective layer 100b and extend upward along the longitudinal axis L such that the second release member 104b may extend past the top edge 112 of the second transducer array 30b and/or past the support layer 14. The second release member 104b and the second protective layer 100b may form a U-shape or a J-shape, for example. In one embodiment, at least a portion of the second release member 104b is positioned adjacent to the first end 105b of the second protective layer 100b.

In one embodiment, removing the protective layer 100 (FIG. 3A), the first protective layer 100a, and/or the second protective layer 100b (FIG. 3B), may be performed after the garment 10 has been placed on the patient 26 and/or after the one or more adjustment assemblies 18 have been adjusted such that the transducer arrays 30 are over the treatment areas and/or target regions of the patient 26. In one embodiment, the support layer 14 may include the aperture 48 (FIG. 1B) through which the release member extends and removing the protective layer 100 may include pulling the release member 104, and thus the protective layer 100 through the aperture 48.

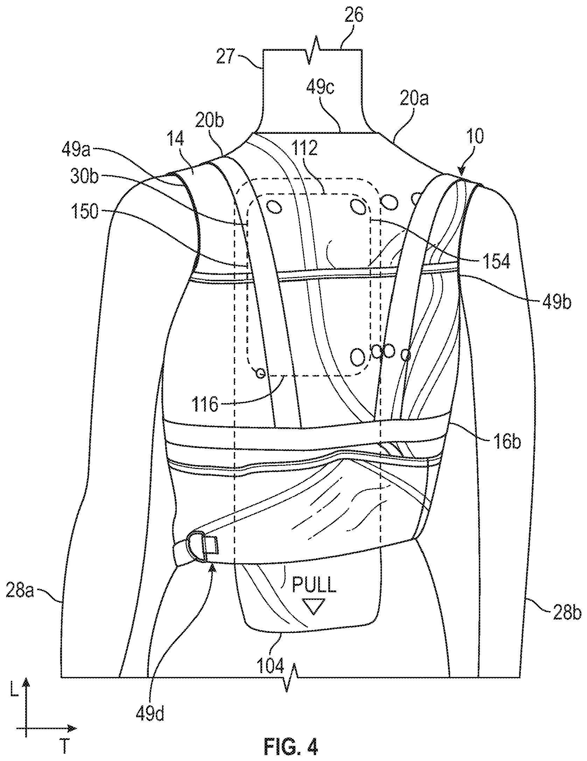

Referring now to FIG. 4, shown therein is a perspective view of an exemplary embodiment of the patient 26 wearing the garment 10 having the second transducer array 30b attached to the back portion 16b of the support layer 14 and the protective layer 100 attached to the second transducer array 30b. The protective layer 100 has the release member 104 shown to extend beyond (in this case, below) the support layer 14 of the garment 10.

While the protective layer 100 and the release member 104 are shown as extending past the first side 150, the second side 154, and top edge 112 of the second transducer array 30b, in other embodiments, the protective layer 100 is coexistent with the first side 150, the second side 154, and the top edge 112. In some embodiments where the second transducer array 30b includes the medical gel on the skin-facing surface 120, the protective layer 100 may extend to cover at least the medical gel but does not necessarily extend as far as edges of the first side 150, the second side 154, or the top edge 112.

While the release members 104, 104a, 104b have generally been described as extending past the support layer 14 or past the garment 10, it is understood that in some embodiments, the release member 104 may extend beyond the second transducer array 30b but may not extend beyond the support layer 14 or the garment 10.

As shown in FIGS. 3A-4, the release member 104 may extend in an orientation of a substantially vertical direction along the longitudinal axis L; however, in other embodiments, the release member 104 may extend in the orientation of a substantially horizontal direction along the transverse axis T. In this embodiment, the release member 104 may be attached to a side other than the second end 108 of the protective layer 100 and may extend in the horizontal direction. In other embodiments, the protective layer 100 and the release members 104, 104a, 104b may have other orientations than vertical and horizontal.

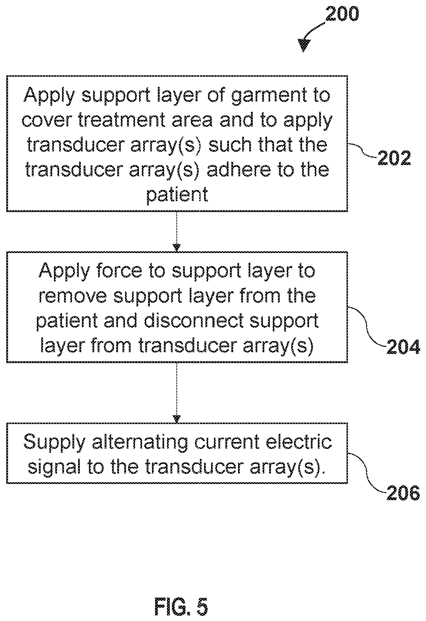

In some implementations a method 200 of use, as shown in FIG. 5, may comprise, applying (step 202) a support layer 14 of the garment 10 to the patient 26 so as to cover a treatment area, and to apply at least one transducer array 30a-n to the patient 26 such that the at least one transducer array 30a-n adheres to the patient 26. The method 200 may further comprise applying (step 204) force to the support layer 14 sufficient to remove the support layer 14 from the patient 26 and disconnect the support layer 14 from the transducer array(s) 30a-n. The method 200 may further comprise supplying (step 206) an alternating current electric signal having a frequency between 50 kHz and 1 MHz to the transducer array(s) 30a-n.

In some implementations, wherein the garment 10 may be a vest having a back portion 16b and at least one arm aperture 49a, and wherein the transducer array 30 may be connected to the back portion 16b of the vest, the step 202 of applying the support layer 14 of the garment 10 to the patient 26 may include receiving the arm 28a of the patient (and/or positioning the arm 28a of the patient 26) through the at least one arm aperture 49a, and drawing (and/or positioning) the back portion 16b of the vest against the back of the patient 26.

In some implementations, the method 200 does not include securing the garment 10 (such as a vest) onto the patient 26 between the step 202 of applying the support layer 14 of the garment 10 to the patient 26 and the step 204 of applying force to the support layer 14 sufficient to remove the support layer 14 from the patient 26. The garment 10 and/or the support layer 14 may be unsecured to the patient 26.

In some implementations, applying at least one transducer array 30a-n to the patient 26 may further include applying at least one transducer array 30a-n having a protective layer, 100, 100a, 100b to the patient. In some implementations, the method 200 may further comprise removing the protective layer 100, 100a, 100b from the at least one transducer array 30a-n after applying the at least one transducer array 30a-n to the patient 26, such that when the protective layer 100, 100a, 100b is removed, the at least one transducer array 30a-n adheres to the patient 26.

In some implementations, the step 204 of applying force to the support layer 14 may be defined further as removing the support layer 14 from the patient 26 such that the transducer array(s) 30a-n remains adhered to the patient 26.

The foregoing description provides illustration and description, but is not intended to be exhaustive or to limit the inventive concepts to the precise form disclosed. Modifications and variations are possible in light of the above teachings or may be acquired from practice of the methodologies set forth in the present disclosure.

From the above description and examples, it is clear that the inventive concepts disclosed and claimed herein are well adapted to attain the advantages mentioned herein. While exemplary embodiments of the inventive concepts have been described for purposes of this disclosure, it will be understood that numerous changes which fall under the scope of the claims may be made.

## Claims

1. A garment, comprising:
a support layer (14) sized and dimensioned to cover at least a portion of a treatment area of a target region;
a first anchor point (20; 20a-n) associated with the support layer (14) and a first position on a patient;
a second anchor point (20; 20a-n) associated with the support layer (14) and a second position on the patient different from the first position;
a transducer array (30; 30a-n) having a skin-facing surface (120) and an opposed, support-layer surface (121), and comprising one or more array fastener (90a'-d') positioned on the support-layer surface (121) and an attaching component (94) positioned on the skin-facing surface (120); and
one or more array fastener (90a-d), complementary to the one or more array fastener (90a'-d') of the transducer array (30; 30a-n), connected to the support layer (14), and being configured to attach to the one or more array fastener (90a'-d') of the transducer array (30; 30a-n) so as to support the transducer array (30; 30a-n) within at least a portion of the treatment area, the one or more array fastener (90a-d) of the support layer (14) and the one or more array fastener (90a'-d') of the transducer array (30; 30a-n) being separable with a first force (126; 126a, b), the transducer array (30; 30a-n) having the attaching component (94) that, when attached to the patient, is separable from the patient with a second force (130; 130a, b) ;
**characterized in that** the second force is
greater than the first force (126; 126a, b), whereby
application of the first force (126; 126a, b) to the transducer array (30; 30a-n) in a direction away from the patient does not cause separation of the transducer array (30; 30a-n) from the patient.

2. The garment of claim 1, further comprising:
a first connection point (22; 22a-n) associated with the support layer (14), the first connection point (22; 22a-n) being disposed at a first vertical position and a first horizontal position of the support layer (14);
a second connection point (22; 22a-n) associated with the support layer (14), the second connection point (22; 22a-n) being disposed at a second vertical position and a second horizontal position of the support layer (14), the second vertical position being vertically displaced from the first vertical position and the second horizontal position being horizontally displaced from the first horizontal position; and
an adjustment assembly (18; 18a-c) adjustably secured to the support layer (14) at the first and second connection points (22; 22a-n) such that adjustment of the adjustment assembly (18; 18a-c) varies spacing between the first and second connection points (22; 22a-n), the adjustment assembly (18; 18a-c) being adjustable between first and second positions, wherein the adjustment assembly (18; 18a-c) in the first position exerts a force (70; 70a-c) in a direction extending from the second connection point (22; 22a-n) to the first connection point (22; 22a-n) and the adjustment assembly (18; 18a-c) is associated with one of the first and second anchor points (20; 20a-n).

3. The garment of claim 2, wherein the adjustment assembly (18; 18a-c) is one or more of a waist strap, a chest strap, a neck strap and a shoulder strap.

4. The garment of any one of claims 1 to 3, wherein the one or more array fastener (90a-d) of the support layer (14) is one or more first array fastener, and further comprising:
a second transducer array (30; 30a-n) having a skin-facing surface (120) and an opposed, support-layer surface (121), and comprising one or more array fastener (90a'-d') positioned on the support-layer surface (121) and an attaching component (94) positioned on the skin-facing surface (120); and
one or more second array fastener (90a-d), complementary to the one or more array fastener (90a'-d') of the second transducer array (30; 30a-n), connected to the support layer (14), and being configured to attach to the one or more array fastener (90a'-d') of the second transducer array (30; 30a-n) within at least a second portion of the treatment area, the one or more second array fastener (90a-d) of the support layer (14) receiving the one or more array fastener (90a'-d') of the second transducer array (30; 30a-n) and having a third force (126; 126a, b) biasing the one or more second array fastener (90a-d) of the support layer (14) and the one or more array fastener (90a'-d') of the second transducer array (30; 30a-n), the second transducer array (30; 30a-n) having the attaching component (94) that, when attached to the patient, has a fourth force (130; 130a, b) biasing the second transducer array (30; 30a-n) towards the patient, wherein the fourth force (130; 130a, b) is greater than the third force (126; 126a, b).

5. The garment of claim 4, wherein the first and second portions of the treatment area substantially coincide.

6. The garment of claim 4, wherein the first and second portions of the treatment area partially coincide.

7. The garment of claim 4, wherein the one or more second array fastener (90a-d) of the support layer (14) includes multiple, spatially-disposed array fasteners (90a-d) configured to be positioned adjacent to the treatment area.

8. The garment of any one of claims 1 to 3, wherein the one or more array fastener (90a-d) of the support layer (14) is selectably associated with the support layer (14).

9. The garment of any one of claims 1 to 3, wherein the transducer array (30; 30an) is attached to the support layer (14) via the one or more array fastener (90a'-d') of the transducer array (30; 30a-n) engaging the one or more array fastener (90a-d) of the support layer (14), and the transducer array (30; 30a-n) being operable to receive a TTField signal and generate a TTField based on the TTField signal.

10. The garment of any one of claims 1 to 3, wherein the one or more array fastener (90a-d) of the support layer (14) and the one or more array fastener (90a'-d') of the transducer array (30; 30a-n) comprise a snap fastener having a snap member and a receiving member configured to receive the snap member.

11. The garment of any one of claims 1 to 3, wherein the one or more array fastener (90a-d) of the support layer (14) and the one or more array fastener (90a'-d') of the transducer array (30; 30a-n) comprise a hook and loop fastener having a hook member and a loop member configured to engage the hook member.

## Patentansprüche

1. Ein Kleidungsstück, bestehend aus:
einer Trägerschicht (14), die so bemessen und dimensioniert ist, dass sie mindestens einen Teil des Behandlungsbereichs einer Zielregion abdeckt;
einem ersten Ankerpunkt (20; 20a-n), der mit der Trägerschicht (14) und einer ersten Position am Patienten verbunden ist;
ein zweiter Ankerpunkt (20; 20a-n), der mit der Trägerschicht (14) verbunden ist und eine zweite Position auf dem Patienten, die sich von der ersten Position unterscheidet;
ein Wandlerarray (30; 30a-n) mit einer der Haut zugewandten Oberfläche (120) und einer gegenüberliegenden Trägerschicht-Oberfläche (121) und mit einem oder mehreren Array-Befestigungselementen (90a'-d'), die auf der Trägerschicht-Oberfläche (121) angeordnet sind, und einer Befestigungskomponente (94), die auf der zur Haut zugewandten Oberfläche (120) angeordnet ist; und
ein oder mehrere Array-Befestigungselemente (90a-d), die komplementär zu dem einen oder den mehreren Array-Befestigungselementen (90a'-d') des Wandlerarrays (30; 30a-n) sind, mit der Trägerschicht (14) verbunden sind und so konfiguriert sind, dass sie an dem einen oder den mehreren Array-Befestigungselementen (90a'-d') des Wandlerarrays (30; 30a-n) befestigt werden, um das Wandlerarray (30; 30a-n) in mindestens einem Teil des Behandlungsbereichs zu halten, wobei der eine oder die mehreren Array-Befestigungselemente (90a-d) der Trägerschicht (14) und das eine oder die mehreren Array-Befestigungselemente (90a'-d') des Wandlerarrays (30; 30a-n) mit einer ersten Kraft (126; 126a, b) trennbar sind, wobei das Wandlerarray (30; 30a-n) die Befestigungskomponente (94) aufweist, die, wenn sie an dem Patienten angebracht ist, mit einer zweiten Kraft (130; 130a, b) von dem Patienten trennbar sind;
**dadurch gekennzeichnet, dass** die zweite Kraft größer ist als die erste Kraft (126; 126a, b), wobei
die Anwendung der ersten Kraft (126; 126a, b) auf das Wandlerarray (30; 30a-n) in einer Richtung weg vom Patienten keine Trennung des Wandlerarrays (30; 30a-n) vom Patienten bewirkt.

2. Das Kleidungsstück gemäß Anspruch 1 umfasst ferner:
einen ersten Verbindungspunkt (22; 22a-n), der mit der Trägerschicht (14) verbunden ist, wobei der erste Verbindungspunkt (22; 22a-n) an einer ersten vertikalen Position und einer ersten horizontalen Position der Trägerschicht (14) angeordnet ist;
einen zweiten Verbindungspunkt (22; 22a-n), welcher der Trägerschicht (14) zugeordnet ist, wobei der zweite Verbindungspunkt (22; 22a-n) an einer zweiten vertikalen Position und einer zweiten horizontalen Position der Trägerschicht (14) angeordnet ist, wobei die zweite vertikale Position gegenüber der ersten vertikalen Position vertikal verschoben ist und die zweite horizontale Position gegenüber der ersten horizontalen Position horizontal verschoben ist; und
eine Verstelleinheit (18; 18a-c), die einstellbar an der Trägerschicht (14) an den ersten und zweiten Verbindungspunkten (22; 22a-n) befestigt ist, so dass die Einstellung der Verstelleinheit (18; 18a-c) den Abstand zwischen den ersten und zweiten Verbindungspunkten (22; 22a-n) verändert, wobei die Verstelleinheit (18; 18a-c) zwischen einer ersten und einer zweiten Position einstellbar ist, wobei die Verstelleinheit (18; 18a-c) in der ersten Position eine Kraft (70; 70a-c) in einer Richtung ausübt, die sich von dem zweiten Verbindungspunkt (22; 22a-n) zu dem ersten Verbindungspunkt (22; 22a-n) erstreckt, und die Verstelleinheit (18; 18a-c) mit einem der ersten und zweiten Ankerpunkte (20; 20a-n) verbunden ist.

3. Das Kleidungsstück gemäß Anspruch 2, wobei es sich bei der Verstelleinheit (18; 18a-c) um einen oder mehrere von einem Taillengurt, einem Brustgurt, einem Nackengurt und einem Schultergurt handelt.

4. Das Kleidungsstück gemäß einem der Ansprüche 1 bis 3, wobei der eine oder die mehreren Array-Befestigungselemente (90a-d) der Trägerschicht (14) ein oder mehrere erste Array-Befestigungselemente sind, und ferner umfassend:
ein zweiter Wandlerarray (30; 30a-n) mit einer der Haut zugewandten Oberfläche (120) und einer gegenüberliegenden Trägerschicht-Oberfläche (121), und mit einem oder mehreren Array-Befestigungselementen (90a'-d'), die auf der Trägerschicht-Oberfläche (121) positioniert sind, und einer Befestigungskomponente (94), die auf der zur Haut zugewandten Oberfläche (120) positioniert ist; und
ein oder mehrere zweite Array-Befestigungselemente (90a-d), die komplementär zu dem einen oder den mehreren Array-Befestigungselementen (90a'-d') des zweiten Wandlerarrays (30; 30a-n) sind, mit der Trägerschicht (14) verbunden sind und so konfiguriert sind, dass sie an dem einen oder den mehreren Array-Befestigungselementen (90a'-d') des zweiten Wandlerarrays (30; 30a-n) innerhalb mindestens eines zweiten Teils des Behandlungsbereichs zu befestigen, wobei das eine oder die mehreren zweiten Array-Befestigungselemente (90a-d) der Trägerschicht (14) das eine oder die mehreren Array-Befestigungselemente (90a'-d') des zweiten Wandlerarrays (30; 30a-n) aufnimmt und eine dritte Kraft (126; 126a, b) aufweist, die das eine oder die mehreren zweiten Array-Befestigungselemente (90a-d) der Trägerschicht (14) und das eine oder die mehreren Array-Befestigungselemente (90a'-d') des zweiten Wandlerarrays (30; 30a-n) vorspannt, wobei das zweite Wandlerarray (30; 30a-n) die Befestigungskomponente (94) aufweist, die, wenn sie an dem Patienten angebracht ist, eine vierte Kraft (130; 130a, b) aufweist, welche das zweite Wandlerarray (30; 30a-n) in Richtung des Patienten vorspannt, wobei die vierte Kraft (130; 130a, b) größer ist als die dritte Kraft (126; 126a, b).

5. Das Kleidungsstück gemäß Anspruch 4, wobei der erste und der zweite Teil des Behandlungsbereichs im Wesentlichen übereinstimmen.

6. Das Kleidungsstück gemäß Anspruch 4, wobei der erste und der zweite Teil des Behandlungsbereichs teilweise übereinstimmen.

7. Das Kleidungsstück gemäß Anspruch 4, wobei das eine oder die mehreren zweiten Array-Befestigungselemente (90a-d) der Trägerschicht (14) mehrere, räumlich angeordnete Array-Befestigungselemente (90a-d) umfassen, die so konfiguriert sind, dass sie angrenzend an den Behandlungsbereich positioniert werden können.

8. Das Kleidungsstück gemäß einem der Ansprüche 1 bis 3, wobei das eine oder mehrere Array-Befestigungselementen (90a-d) der Trägerschicht (14) wahlweise mit der Trägerschicht (14) verbunden ist.

9. Das Kleidungsstück gemäß einem der Ansprüche 1 bis 3, wobei das Wandlerarray (30; 30a-n) an der Trägerschicht (14) über das eine oder die mehreren Array-Befestigungselemente (90a'-d') des Wandlerarrays (30; 30a-n) angebracht ist, die in das eine oder die mehreren Array-Befestigungselemente (90a-d) der Trägerschicht (14) eingreifen, und wobei das Wandlerarray (30; 30a-n) so betrieben werden kann, dass es ein TTFeld-Signal empfängt und ein TTFeld basierend auf dem TTFeld-Signal erzeugt.

10. Das Kleidungsstück gemäß einem der Ansprüche 1 bis 3, wobei das eine oder die mehreren Array-Befestigungselemente (90a-d) der Trägerschicht (14) und das eine oder die mehreren Array-Befestigungselemente (90a'-d') des Wandler-Arrays (30; 30a-n) einen Druckknopf mit einem Druckteil und einem Aufnahmeelement umfassen, das zur Aufnahme des Druckteils konfiguriert ist.

11. Das Kleidungsstück gemäß einem der Ansprüche 1 bis 3, wobei das eine oder die mehreren Array-Befestigungselemente (90a-d) der Trägerschicht (14) und das eine oder die mehreren Array-Befestigungselemente (90a'-d') des Wandlerarrays (30; 30a-n) einen Klettverschluss mit einem Hakenband und einem Schlaufenband umfassen, die so konfiguriert sind, dass sie mit dem Hakenband in Eingriff kommen.

## Revendications

1. Vêtement qui comprend :
une couche de support (14) dimensionnée pour couvrir au moins une partie d'une zone de traitement d'une région cible ;
un premier point d'ancrage (20 ; 20a-n) lié à la couche de support (14) et à une première position sur un patient ;
un deuxième point d'ancrage (20 ; 20a-n) lié à la couche de support (14) et à un deuxième endroit sur le patient différent du premier endroit ;
un réseau de transducteurs (30 ; 30a-n) avec une surface (120) qui est face à la peau et une surface opposée (121) qui est une couche de support, et qui comprend un ou plusieurs éléments de fixation du réseau (90a'-d') placés sur la surface de la couche de support (121) et un composant de fixation (94) placé sur la surface (120) qui est face à la peau ; et
un ou plusieurs éléments de fixation de réseau (90a-d), qui vont bien avec le ou les éléments de fixation de réseau (90a'-d') du réseau de transducteurs (30 ; 30a-n), reliés à la couche de support (14) et conçus pour se fixer au ou aux éléments de fixation de réseau (90a'-d') du réseau de transducteurs (30 ; 30a-n) de manière à supporter le réseau de transducteurs (30 ; 30a-n) à l'intérieur d'au moins une partie de la zone de traitement, la ou les fixations de réseau (90a-d) de la couche de support (14) et la ou les fixations de réseau (90a'-d') du réseau de transducteurs (30 ; 30a-n) pouvant être séparés par une première force (126; 126a, b), le réseau de transducteurs (30 ; 30a-n) ayant le composant de fixation (94) qui, lorsqu'il est fixé au patient, peut être séparé du patient par une deuxième force (130 ; 130a, b) ;
**caractérisé en ce que** la deuxième force est plus grande que la première force (126 ; 126a, b), ce qui fait que
l'application de la première force (126 ; 126a, b) sur le réseau de transducteurs (30 ; 30a-n) dans une direction s'éloignant du patient ne provoque pas la séparation du réseau de transducteurs (30 ; 30a-n) du patient.

2. Procédé selon la revendication 1, comprenant en outre :
un premier point de connexion (22 ; 22a-n) associé à la couche de support (14), le premier point de connexion (22 ; 22a-n) étant placé à une première position verticale et à une première position horizontale de la couche de support (14) ;
un deuxième point de connexion (22 ; 22a-n) associé à la couche de support (14), le deuxième point de connexion (22 ; 22a-n) étant placé à une deuxième position verticale et à une deuxième position horizontale de la couche de support (14), la deuxième position verticale étant décalée verticalement par rapport à la première position verticale et la deuxième position horizontale étant décalée horizontalement par rapport à la première position horizontale ; et
un ensemble de réglage (18 ; 18a-c) fixé de manière réglable à la couche de support (14) au niveau des premier et deuxième points de connexion (22 ; 22a-n) de manière à ce que le réglage de l'ensemble de réglage (18 ; 18a-c) fasse varier l'espacement entre les premier et deuxième points de connexion (22 ; 22a-n), l'ensemble de réglage (18 ; 18a-c) étant réglable entre des première et deuxième positions, dans lequel l'ensemble de réglage (18 ; 18a-c) dans la première position exerce une force (70 ; 70a-c) dans une direction s'étendant du deuxième point de connexion (22 ; 22a-n) vers le premier point de connexion (22 ; 22a-n) et l'ensemble de réglage (18 ; 18a-c) est associé à l'un des premier et deuxième points d'ancrage (20 ; 20a-n).

3. Le vêtement de la revendication 2, où l'ensemble de réglage (18 ; 18a-c) est une ou plusieurs des sangles suivantes : une sangle de taille, une sangle de poitrine, une sangle de cou et une sangle d'épaule.

4. Vêtement selon l'une quelconque des revendications 1 à 3, dans lequel la ou les attaches en réseau (90a-d) de la couche de support (14) sont une ou plusieurs premières attaches en réseau, et comprenant en plus :
un deuxième réseau de transducteurs (30 ; 30a-n) avec une surface (120) qui est face à la peau et une surface opposée (121) qui est une couche de support, et qui comprend un ou plusieurs éléments de fixation (90a'-d') pour le réseau, placés sur la surface (121) de la couche de support, et un composant de fixation (94) placé sur la surface (120) qui est face à la peau ; et
un ou plusieurs seconds éléments de fixation de réseau (90a-d), complémentaires du ou des éléments de fixation de réseau (90a'-d') du second réseau de transducteurs (30 ; 30a-n), reliés à la couche de support (14) et configurés pour se fixer au ou aux éléments de fixation de réseau (90a'-d') du second réseau de transducteurs (30 ; 30a-n) à l'intérieur d'au moins une deuxième partie de la zone de traitement, la ou les deuxièmes attaches de réseau (90a-d) de la couche de support (14) recevant la ou les attaches de réseau (90a'-d') du deuxième réseau de transducteurs (30 ; 30a-n) et ayant une troisième force (126 ; 126a, b) qui fait pencher la ou les deuxièmes attaches de réseau (90a-d) de la couche de support (14) et la ou les attaches de réseau (90a'-d') du deuxième réseau de transducteurs (30 ; 30a-n), le deuxième réseau de transducteurs (30 ; 30a-n) ayant le composant de fixation (94) qui, lorsqu'il est fixé au patient, a une quatrième force (130 ; 130a, b) qui tire le deuxième réseau de transducteurs (30 ; 30a-n) vers le patient, où la quatrième force (130 ; 130a, b) est plus grande que la troisième force (126 ; 126a, b).

5. Le vêtement de la revendication 4, où les première et deuxième parties de la zone de traitement se rejoignent à peu près.

6. Le vêtement de la revendication 4, où les première et deuxième parties de la zone de traitement se rejoignent un peu.

7. Le vêtement de la revendication 4, où la ou les deuxièmes attaches en réseau (90ad) de la couche de support (14) ont plusieurs attaches en réseau (90a-d) placées de manière à être à côté de la zone de traitement.

8. Vêtement selon l'une quelconque des revendications 1 à 3, dans lequel la ou les attaches en réseau (90a-d) de la couche de support (14) sont associées de manière sélective à la couche de support (14).

9. Vêtement selon l'une quelconque des revendications 1 à 3, dans lequel le réseau de transducteurs (30 ; 30a-n) est fixé à la couche de support (14) par l'intermédiaire d'un ou plusieurs éléments de fixation de réseau (90a'-d') du réseau de transducteurs (30 ; 30an) s'engageant dans la ou les attaches de réseau (90a-d) de la couche de support (14), et le réseau de transducteurs (30 ; 30a-n) pouvant être actionné pour recevoir un signal TTField et générer un TTField basé sur le signal TTField.

10. Vêtement selon l'une quelconque des revendications 1 à 3, dans lequel la ou les attaches en réseau (90a-d) de la couche de support (14) et la ou les attaches en réseau (90a'-d') du réseau de transducteurs (30 ; 30a-n) comprennent une attache à pression comportant un élément de pression et un élément de réception configuré pour recevoir l'élément de pression.

11. Vêtement selon l'une quelconque des revendications 1 à 3, dans lequel la ou les attaches en réseau (90a-d) de la couche de support (14) et la ou les attaches en réseau (90a'-d') du réseau de transducteurs (30 ; 30a-n) comprennent une attache à crochets et boucles comportant un élément à crochets et un élément à boucles configuré pour s'engager avec l'élément à crochets.
